# EUROPEAN PATENT SPECIFICATION

(11) **EP 4 522 076 B1**
(45) Date of publication and mention of the grant of the patent: **08.04.2026**
(21) Application number: 23726081.5
(22) Date of filing: 10.05.2023
(51) Int. Cl.: A61F 2/24

(54) **ANNULOPLASTY DEVICE**
ANNULOPLASTIEVORRICHTUNG
DISPOSITIF D'ANNULOPLASTIE

(30) Priority: 11.05.2022 US 202263340703 P
(43) Date of publication of application: 19.03.2025
(73) Proprietor: HVR Cardio Oy, 02600 Espoo (FI)
(72) Inventor: KERÄNEN, Olli, 23741 Bjärred (SE)
(74) Representative: Invent Horizon IP
(86) International application number: PCT/EP2023/062524
(87) International publication number: WO 2023/217922

(56) References cited:
- EP-A1- 3 766 457
- WO-A1-2022/012782
- US-A1- 2010 049 315
- US-A1- 2021 177 597

## Description

### Field of the Invention

This invention pertains in general to the field of cardiac valve repair. More particularly the invention relates to an annuloplasty device, such as an annuloplasty ring or helix, for positioning at the heart valve annulus. A method of repairing a defective heart valve is disclosed.

### Background of the Invention

Diseased mitral and tricuspid valves frequently need replacement or repair. The mitral and tricuspid valve leaflets or supporting chordae may degenerate and weaken or the annulus may dilate leading to valve leak. Mitral and tricuspid valve replacement and repair are frequently performed with aid of an annuloplasty ring, used to reduce the diameter of the annulus, or modify the geometry of the annulus in any other way, or aid as a generally supporting structure during the valve replacement or repair procedure. The annuloplasty ring is typically implanted around the annulus of the heart valve.

A problem with prior art annuloplasty implants is to achieve correct positioning at the heart valve and fixate the implant in the correct position. Suturing devices for annuloplasty implants have disadvantages that makes it difficult to suture in the correct position, thereby resulting insufficient suturing strength, and also in a very time-consuming procedure, which increases the risks for the patient. Furthermore, suturing devices are often not sufficiently compact for catheter based procedures. The use of clips for positioning annuloplasty implants is also associated with challenges, in particular when implanting helix rings that are to be positioned on either side of a heart valve. Insufficient fixation of such implant lead to traumatic effects since the fixation structure must ensure the correct position of the device over time. A further problem in the prior art is thus also to achieve a reliable fixation at the annulus of the heart valve. An annuloplasty implant is intended to function for years and years, so it is critical with long term stability in this regard.

The above problems may have dire consequences for the patient and the health care system. Patient risk is increased. WO2022/012782A1 discloses first and second support rings and a stent positioned onto the first and second support rings. The stent has retention units. EP3766457A1 discloses an annuloplasty device having retention units with a retracted position and an expanded position. An elongated element can actuate the retention units to provide the expanded position.

Hence, an improved annuloplasty implant or device would be advantageous and in particular allowing for avoiding more of the above mentioned problems and compromises, and in particular ensuring secure fixation of the annuloplasty device, during the implantation phase, and for long-term functioning, in addition to a less complex procedure, and increased patient safety. A related method would also be advantageous.

### Summary of the Invention

Accordingly, examples of the present invention preferably seek to mitigate, alleviate or eliminate one or more deficiencies, disadvantages or issues in the art, such as the above-identified, singly or in any combination by providing a device according to the appended patent claims.

According to a first aspect an annuloplasty device is provided comprising first and second support rings having a coiled configuration in which the first and second support rings are arranged as a coil around a central axis, wherein the first and second support rings are configured to be arranged on opposite sides of native heart valve leaflets of a heart valve, a stent arranged around at least a portion of the first and/or second support ring, and wherein the stent comprises retention units.

Further examples of the invention are defined in the dependent claims.

Some examples of the disclosure provide for a facilitated positioning of an annuloplasty device at a heart valve.

Some examples of the disclosure provide for a facilitated fixation of an annuloplasty device at a heart valve.

Some examples of the disclosure provide for a less time-consuming fixation of an annuloplasty to a target site.

Some examples of the disclosure provide for securing long-term functioning and position of an annuloplasty device.

Some examples of the disclosure provide for a reduced risk of damaging the anatomy of the heart such as the annulus or the valve leaflets.

Some examples of the disclosure provide for a more secure implantation of an annuloplasty device in narrow anatomies.

It should be emphasized that the term "comprises/comprising" when used in this specification is taken to specify the presence of stated features, integers, steps or components but does not preclude the presence or addition of one or more other features, integers, steps, components or groups thereof.

### Brief Description of the Drawings

These and other aspects, features and advantages of which embodiments of the invention are capable of will be apparent and elucidated from the following description of embodiments of the present invention, reference being made to the accompanying drawings, in which
Fig. 1 is a schematic illustration of an annuloplasty device, in a perspective view, where stents are arranged around at least a portion of the first and second support rings, according to an example of the disclosure;
Fig. 2 is a schematic illustration of a stent of the annuloplasty device, where the stent has a plurality of retention units, according to an example of the disclosure;
Fig. 3 is a schematic illustration of a portion of a stent of the annuloplasty device, in a further detailed view, according to an example of the disclosure;
Fig. 4a is a schematic illustration of a portion of a stent of the annuloplasty device, in detailed view, where a retention unit thereof is in a retracted state, according to an example of the disclosure;
Fig. 4b is a schematic illustration of a portion of a stent of the annuloplasty device, in detailed view, where a retention unit thereof is in an expanded state, according to an example of the disclosure;
Fig. 4c is a schematic illustration of a portion of a stent of the annuloplasty device, in front view of the retention unit thereof, where the retention unit is in a retracted state, according to an example of the disclosure;
Fig. 4d is a schematic illustration of a portion of a stent of the annuloplasty device, in detailed view, where a retention unit thereof is in a retracted state, according to an example of the disclosure;
Fig. 4e is a schematic illustration of a portion of a stent of the annuloplasty device, in detailed view, where a retention unit thereof is in an expanded state, according to an example of the disclosure;
Fig. 4f is a schematic illustration of a portion of a stent of the annuloplasty device, in detailed view, where a retention unit thereof is in a retracted state, according to an example of the disclosure;
Fig. 4g is a schematic illustration of a portion of a stent of the annuloplasty device, in detailed view, where a retention unit thereof is in an expanded state, according to an example of the disclosure;
Fig. 4h is a schematic illustration of a portion of a stent of the annuloplasty device, in detailed view, where a retention unit thereof is in an expanded state, according to an example of the disclosure;
Fig. 4i is a schematic illustration of the annuloplasty device in Fig. 4h, in a cross-sectional view, where a retention unit of a stent is in an expanded state, according to an example;
Figs. 5a-b are schematic illustrations of the annuloplasty device, in cross-sectional views, where a retention unit of a stent is in an expanded state, according to examples of the disclosure;
Fig. 6 is a schematic illustration of the annuloplasty device, in side-view, where first and second support rings are separated by a transition region, according to examples of the disclosure;
Fig. 7a is a schematic illustration of an annuloplasty device, in a side view, where the annuloplasty device is positioned above and below valve leaflets, according to an example of the disclosure;
Fig. 7b is a schematic illustration of an annuloplasty device, in a top-down view, according to an example of the disclosure;
Figs. 8a-b are schematic illustration of a stent of the annuloplasty device, where the stent is radially expanded (a) and radially retracted (b), respectively, according to an example of the disclosure;
Figs. 9a-b are schematic illustration of a stent of the annuloplasty device, where the stent is radially expanded (a) and radially retracted (b), respectively, according to an example of the disclosure;
Figs. 10a-d are schematic illustrations of a stent of the annuloplasty device, where the stent has a plurality of retention units, according to examples of the disclosure;
Fig. 11a is a flow chart of a method of repairing a defective heart valve, according to an example of the disclosure;
Fig. 11b is another flow chart of a method of repairing a defective heart valve, according to an example of the disclosure;
Figs. 12a-c are schematic illustrations of a section of a stent of the annuloplasty device, where the stent has a plurality of retention units, according to examples of the disclosure;
Fig. 13 is a schematic illustration of a section of a stent of the annuloplasty device, where the stent has a plurality of retention units, according to an example;
Figs. 14a-d are schematic illustrations of a retention units of a stent of the annuloplasty device, according to examples of the disclosure;
Figs. 15a-b are schematic illustrations of a stent of the annuloplasty device, in a perspective view (15a) and in a side view (15b), where the stent has a plurality of retention units, according to examples of the disclosure; and
Figs. 16a-b are schematic illustrations of a stent of the annuloplasty device, in a perspective view (15a) and in a side view (15b), where the stent has a plurality of retention units, according to examples of the disclosure.

### Description of embodiments

Specific embodiments of the invention will now be described with reference to the accompanying drawings. This invention may, however, be embodied in many different forms and should not be construed as limited to the embodiments set forth herein; rather, these embodiments are provided so that this disclosure will be thorough and complete, and will fully convey the scope of the invention to those skilled in the art. The terminology used in the detailed description of the embodiments illustrated in the accompanying drawings is not intended to be limiting of the invention. In the drawings, like numbers refer to like elements.

The following description focuses on an embodiment of the present invention applicable to cardiac valve implants such as annuloplasty rings. However, it will be appreciated that the invention is not limited to this application but may be applied to many other annuloplasty implants and cardiac valve implants including for example replacement valves, and other medical implantable devices.

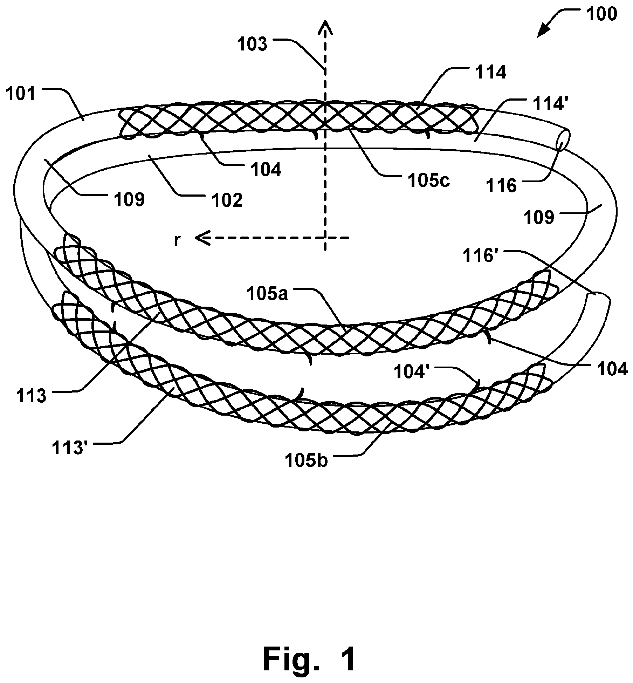

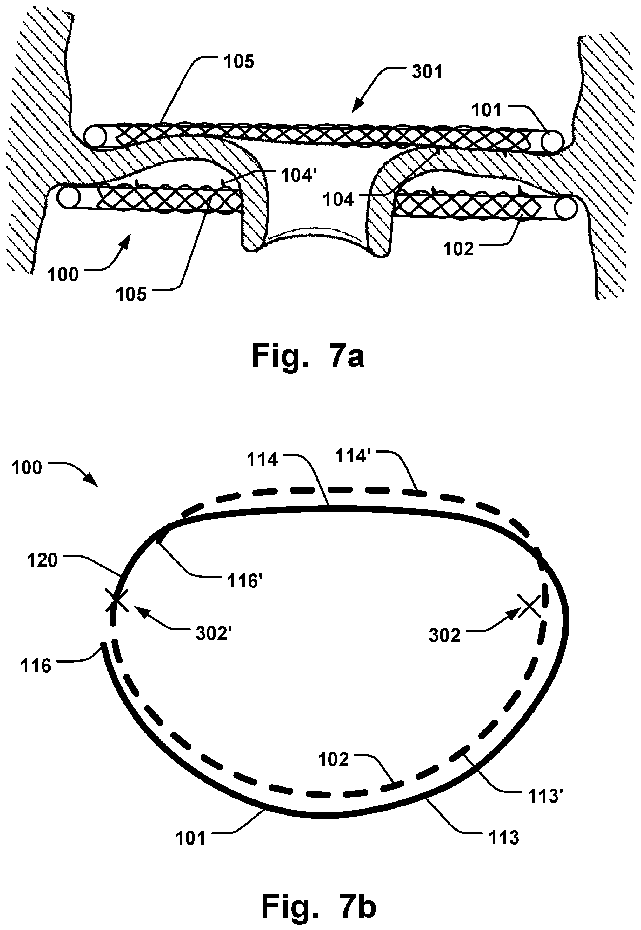

Fig. 1 schematically illustrates an example of an annuloplasty device 100 comprising a first support ring 101 and second support ring 102 which are adapted to be arranged as a coil, i.e. in a helix-shape, in a coiled configuration around a central axis 103, as illustrated in Fig. 1. The device 100 is arranged in the coiled configuration at least when in a relaxed state of the material from which the device 100 is formed, i.e. free from outside forces acting upon the device 100. The coil-shaped device 100 has two free ends 116, 116'. The first and second support rings 101, 102, and the respective free ends 116, 116', are configured to be arranged on opposite sides of native heart valve leaflets 301 of a heart valve, as illustrated in the side view of Fig. 7a. As shown in Fig. 7a, the first support ring 101 may be arranged on an atrial side of the heart valve, and the second support ring 102 may be arranged on a ventricular side (the second support ring 102 is also shown with dashed lines in the top-down view of Fig. 7b, where the valve leaflets have been omitted). The second support ring 102 is illustrated with a dashed line and is in these examples arranged on the ventricular side of the heart valve, whereas the first support ring 101 is arranged on the atrial side of the heart valve (shown with solid line in Fig. 7b). The first support ring 101 may thus extend along the annulus of the heart valve on the atrial side. The first and second support rings 101, 102, are connected to form a coil- or helix shaped ring, as an integral continuous ring. The coil extends through the valve opening at a commissure 302, 302', thereof. In the example of Fig. 7b, the coil extends through commissure denoted as 302', but is should be understood that the annuloplasty device 100 may extend through the commissure denoted as 302 in other examples. The first and second support rings 101, 102, may thus assume the coiled configuration also when in an implanted state. As explained further below, the device 100 may comprise a shape-memory material, so that the device 100 re-assumes the coiled configuration after having been delivered from a catheter (not shown) to the target site, after having been temporarily restrained in an elongated configuration of the catheter. The annuloplasty device 100, i.e. annuloplasty implant 100, may comprise a shape memory material, such as NiTiNol, or another suitable biocompatible alloy that can be heat-set in defined shapes, i.e. in a defined relaxed shape in absence of outside acting forces, in a heat treatment procedure. The annuloplasty device 100 may pinch the tissue of the valve leaflets 301, between the first and second support rings 101, 102, i.e. with forces acting in parallel with the central axis 103.

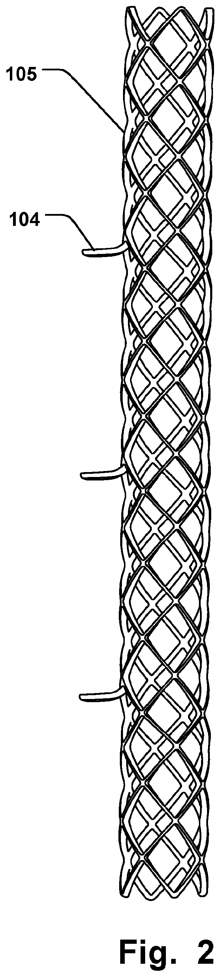

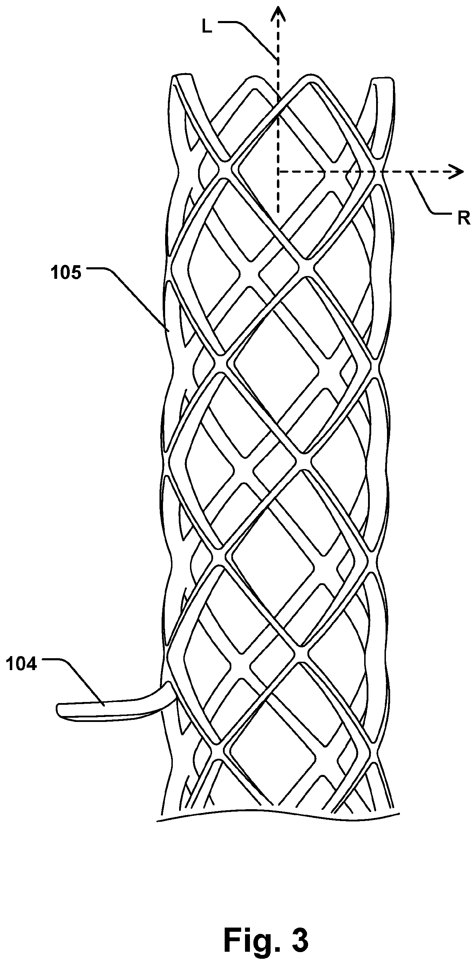

The annuloplasty device 100 further comprises a stent 105, 105a, 105b, 105c, arranged around at least a portion of the first and/or second support ring 101, 102. Fig. 1 shows an example where three stents 105a, 105b, 105c, are arranged around portions of the first and second support rings 101, 102. Figs. 2 - 4 are further detailed views of a stent 105 configured to be arranged around at least a portion of the first and/or second support ring 101, 102. It should be understood that the annuloplasty device 100 may comprise a varying number of stents 105 depending on the particular implant site of the annuloplasty device 100. Furthermore, the ratio of the total length of the first and/or second support ring 101, 102, covered by the stent 105, 105a, 105b, 105c, may vary depending on the placement of the annuloplasty device 100. Although reference is made to stent 105 in the present disclosure, it should be understood that any of the stents 105a, 105b, 105c, as exemplified in Fig. 1 may comprise the features as described for stent 105 in relation to Figs. 2 - 10. The lattice or framework of the stent 105 may be formed by laser cutting of a tube-shaped material, such as NiTinol or other bio compatible metal alloy and then pushed over the first and/or second support rings 101, 102. The stent 105 thus has a hollow interior to accommodate the first and/or second support rings 101, 102. The stent comprises retention units 104, 104', as schematically illustrated in Fig. 1 and in the detailed views of Figs. 3 - 5, 10. The retention units 104, 104', are shaped to pierce into tissue at the heart valve. The retention units 104, 104', are fixed in relation to the stent 105, and the stent 105 is fixed in relation to the first and/or second support ring 101, 102, on which the stent 105 is arranged. Thus, having stents 105a, 105b, 105c, arranged around at least part of the first and/or second support rings 101, 102, provides for anchoring the annuloplasty device 100 to the valve tissue with the retention units 104, 104'. The first and/or second support rings 101, 102, are thus provided with a robust anchoring mechanism by utilizing a stent 105, 105a, 105b, 105c, as an intermediate fixation structure for the retention units 104, 104', thereby dispensing with the need to attach any retention structures directly to the first and/or second support rings 101, 102. The stent 105 thus provides for increasing the reliability of the anchoring mechanism of the annuloplasty device 100 as the number of separate structures needing to be joined together can be reduced, in particular in the example where the retention units 104, 104', are integrated with the stent 105 as mentioned below. Long-term reliability of the annuloplasty device 100 may thus be improved. The manufacturing of the annuloplasty device 100 may thus also be facilitated, as the number of separate elements is minimized. Manufacturing tolerances may thus be easier to comply with and the overall complexity and associated costs may be reduced, providing for a more viable annuloplasty implant 100. Having an annuloplasty device 100 with stents 105, 105a, 105b, 105c, and associated retention units 104, 104', also provides for a modular annuloplasty device 100 where a core structure of the first and second support rings 101, 102, may be provided with a stents 105, 105a, 105b, 105c, having retention units 104, 104', in varying configurations and shapes depending on the particular application. The annuloplasty device 100 may thus be tailored to the particular patient and anatomical circumstances more easily and patient safety can be further improved.

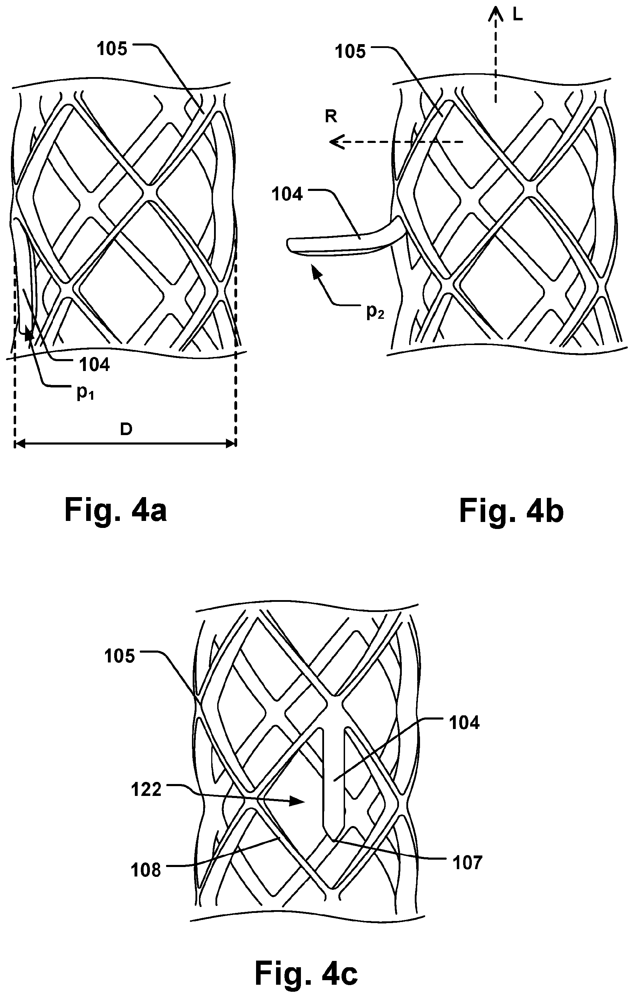

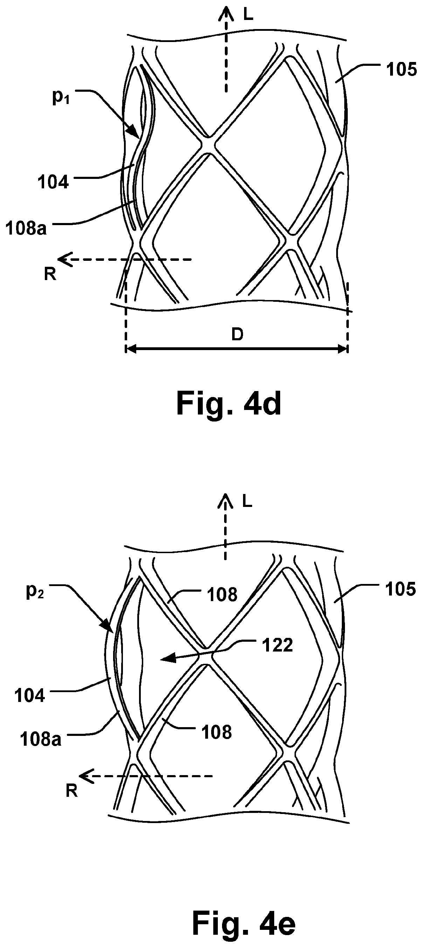

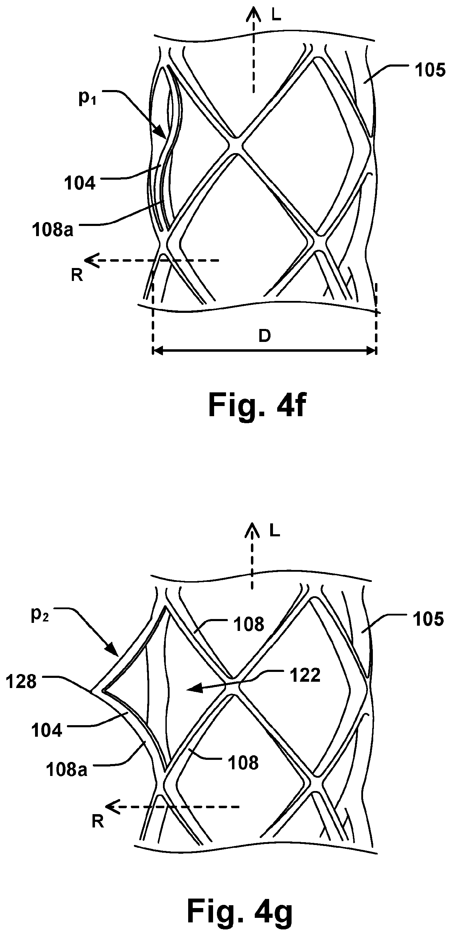

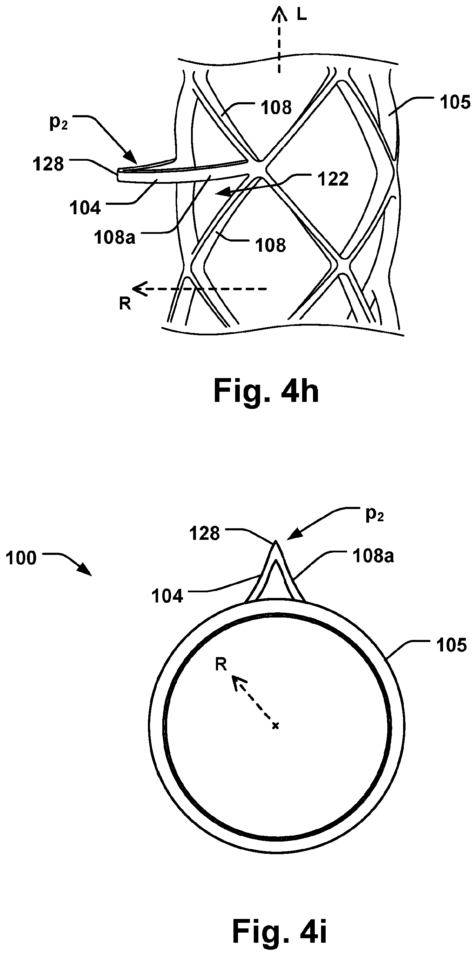

As elucidated above, the retention units 104, 104', may be formed from the material of the stent 105. The retention units 104, 104', may thus be integrated with the stent 105. The detailed views in e.g. Fig. 4c, 4e, 4g, or 4h are schematic examples of how retention unit 104 is formed as a part of the framework of the stent 105. The retention unit 104 may be cut as an elongated structure with a free tip 107 within the structural framework of the stent 105, as exemplified in Fig. 4c. **In** the example of Fig. 4c, the retention unit 104 is surrounded by support elements 108 of the stent 105. The retention unit 104 with an elongated structure having a free tip 107 may be movable along a radial direction (R), perpendicular to a longitudinal direction (L) of the stent 105, from a retracted state (p₁) (Fig. 4a), to an expanded state (p₂) (Fig. 4b), as described further below.

The examples in Figs. 2 - 4 show support elements 108 arranged in a rhombic pattern or closed cells 122, where the retention units 104 extend into the void of individual rhombs or cells 122 at defined positions along the length of the stent 105. The stent 105 may thus comprise a plurality of support elements 108 forming a stent framework of closed cells 122, as further schematically illustrated in the examples shown in Figs. 4d-h. A first support element 108a of the plurality of support elements 108 of a cell 122 may be movable as a retention unit 104, 104', along a radial direction (R), perpendicular to a longitudinal direction (L) of the stent 105, as schematically illustrated in the examples of Figs. 4d-e, 4f-g and 4h-i. The retention unit 104 illustrated in Figs. 4d-i may thus be part of the support elements 108 forming a closed cell 122. Figs. 4d-e show the retention unit 104, i.e. the first support element 108a, arranged in a retracted state (p₁) (Fig. 4d), and in an expanded state (p₂) (Fig. 4e), respectively. The retention unit 104, or first support element 108a, may be expanded like a bow-like structure in the radial direction (R) in the expanded state (p₂). The bow-like shape of the first support element 108a may thus be configured to apply a pressure into the valve tissue and increase the retention force of the stent 105 at the annulus. The retention unit 104, or first support element 108a, may be folded into an undulated or otherwise curved or folded shape in the retracted state (p₁), as exemplified in Fig. 4d. Figs. 4f-g show the retention unit 104, i.e. the first support element 108a, arranged in a retracted state (p₁) (Fig. 4f), and in an expanded state (p₂) (Fig. 4g), respectively. The retention unit 104, or first support element 108a, may be expanded as an angular structure with an apex point 128 extending furthest away from the stent 105 in the radial direction (R). The apex point 128 may engage the tissue to create a retention force for the stent 105. The apex point 128 may be sufficiently sharp to penetrate into the tissue. The retention unit 104, or first support element 108a, may thus form a V-shape, or an essentially triangular shape with an imaginary base extending across the width of the cell 122. The stent 105 may comprise a plurality of such retention units 104 along the longitudinal direction (L). The retention unit 104, or first support element 108a in Fig. 4g may be folded into an undulated or otherwise curved or folded shape in the retracted state (p₁), as exemplified in Fig. 4f.

The support element 108a, or at least a projection thereof on the generally cylindrical surface formed by the framework of the stent 105, may extend essentially along the longitudinal direction (L) of the stent 105, at least when in the expanded state (p₂), as shown in the example of Fig. 4g. The example in Fig. 4h shows the support element 108a, or at least a projection thereof on the generally cylindrical surface formed by the framework of the stent 105, extending essentially perpendicular to the longitudinal direction (L) of the stent 105, when the expanded state (p₂). The retention unit 104, or first support element 108a, may be expanded to an angular structure, such as a V-shape as described above, with an apex point 128 extending furthest away from the stent 105 in the radial direction (R), as schematically illustrated in Fig. 4h. The retention unit 104, or first support element 108a in Fig. 4h may be folded into an undulated or otherwise curved or folded shape in the retracted state (p₁) (not shown). Fig. 4i shows a cross-sectional view of the stent 105 in Fig. 4h along the longitudinal direction (L), being rotated in Fig. 4i so that the retention unit 104, or first support element 108a, points vertically upward. The retention unit 104, or first support element 108a, is in the expanded state (p₂) as described above. The stent 105 may comprise a plurality of such retention units 104 along the longitudinal direction (L).

It should be understood the support elements 108 may be cut to form varying patterns. Forming the retention units 104, 104', as integrated structures of the framework of the stent 105 provides for robust and strong retention units 104, 104', and a minimized risk of dislocations or deformations thereof over time. An overall robust and reliable fixation mechanism of the annuloplasty device 100 is thus provided. Manufacturing is also facilitated, as mentioned above, as the number of separate elements of the annuloplasty device 100 requiring assembly is minimized. The retention units 104, 104', may be cut to form various shapes for optimizing the gripping force into the tissue. The retention units 104, 104', may be formed by different cutting techniques such as by laser cutting techniques.

The retention units 104, 104', may be heat-set to assume a defined bent or angled shape as schematically illustrated in the examples of Figs. 4b, 4e, 4g, 4h or 4i, showing an expanded state (p₂) of the retention unit 104. The expanded state (p₂) may thus correspond to a relaxed state of the retention unit 104 where the latter is not acted upon by external forces. The retention unit 104 may be bent and heat-treated during manufacturing so that the retention unit 104 assumes a defined shape in the expanded state (p₂). The retention unit 104 may thus have a bias towards the expanded state (p₂), by striving towards the relaxed expanded state (p₂).

The retention units 104, 104', may thus be resiliently moveable from a retracted state (p₁) to the expanded state (p₂). For example, a force may be applied to the retention unit 104 so that it bends and assumes a retracted position or state (p₁), as exemplified in Fig. 4a, 4d, or 4f, e.g. if a delivery catheter (not shown) applies a compressive force onto the stent 105 and the related retention unit 104. As the stent 105 is ejected from the delivery catheter, when the annuloplasty device 100 is deployed from the delivery catheter, the compressive force is removed and the resilience of the retention unit 104 cause it to move towards the expanded state (p₂). This provides for an effective deployment of the retention units 104, 104', as the first and second support rings 101, 102, of the annuloplasty device 100 are ejected from the delivery catheter. The retention units 104, 104', can thus expand and pierce into the valve tissue. The cross-section of the annuloplasty device 100 may be minimized as the retention units 104, 104', may assume the retracted state (p₁) when positioned inside the delivery catheter. A smaller cross-section provides for a facilitated navigation of the annuloplasty device 100 to a target site in the heart. The delivery catheter may also be subject to less abrasion and wear from the retention units 104, 104', as these may assume the retracted state (p₁) inside the delivery catheter, causing less friction between the tip 107 and the inside lumen of the delivery catheter. Reduced friction also facilitates moving the annuloplasty device 100 along the delivery catheter, requiring less force and improving the amount of control.

Hence, the retention units 104, 104', may be flexible to bend from the expanded state (p₂) to the retracted state (p₁). This allows also for the retention units 104, 104', to flex to the retracted state (p₁) if withdrawing the annuloplasty device 100 into a delivery catheter, in case the implantation is aborted or repositioning is needed. The annuloplasty device 100 may thus re-assume the compact cross-sectional profile.

In one example the retention units 104, 104', may comprise a shape-memory material, where activation of the shape-memory material causes the retention units 104, 104', to transfer from the retracted state (p₁) to the expanded state (p₂). For example, the shape-memory material may be temperature activated, so that the retention units 104, 104', move towards the expanded state (p₂) when subject to heating to the body temperature. This provides for an advantageous deployment of the retention units 104, 104', in some applications.

The retention units 104, 104', may be aligned essentially flush with an outer diameter (D) of the stent 105 in the retracted state (p₁), as schematically illustrated in Fig. 4a, 4d, or 4f. This provides for a compact cross-sectional profile of the annuloplasty device 100 as well as reduced risk of high pressure and abrasion of the retention units 104, 104', against an inner lumen of a delivery catheter.

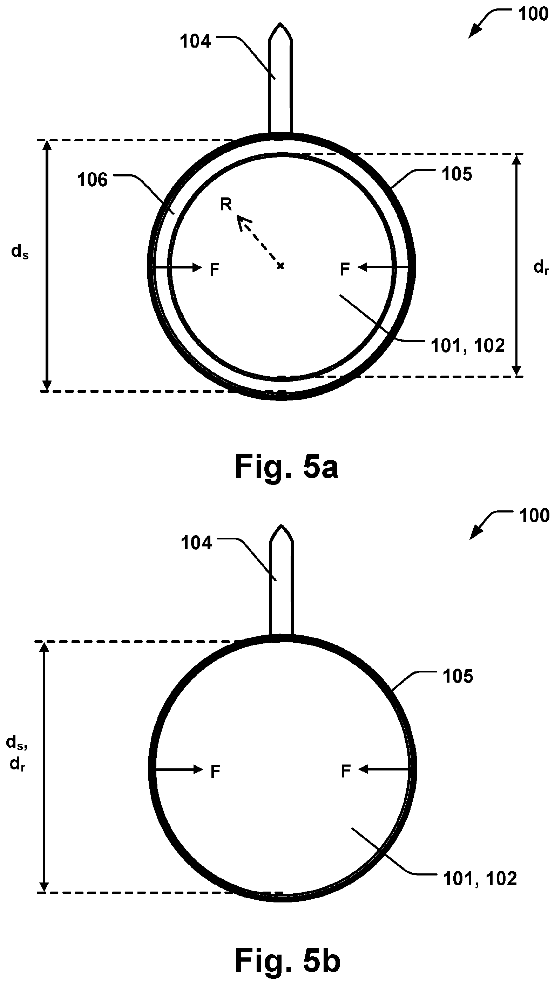

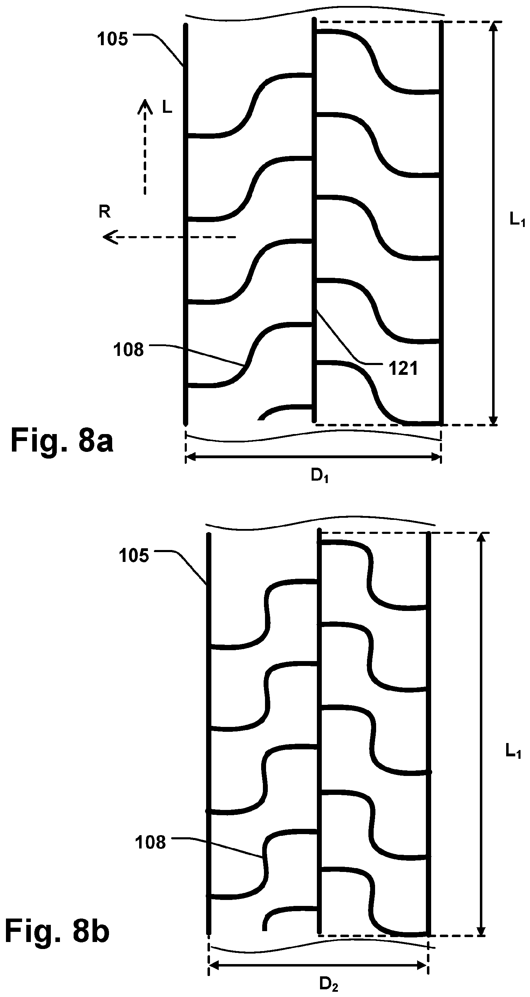

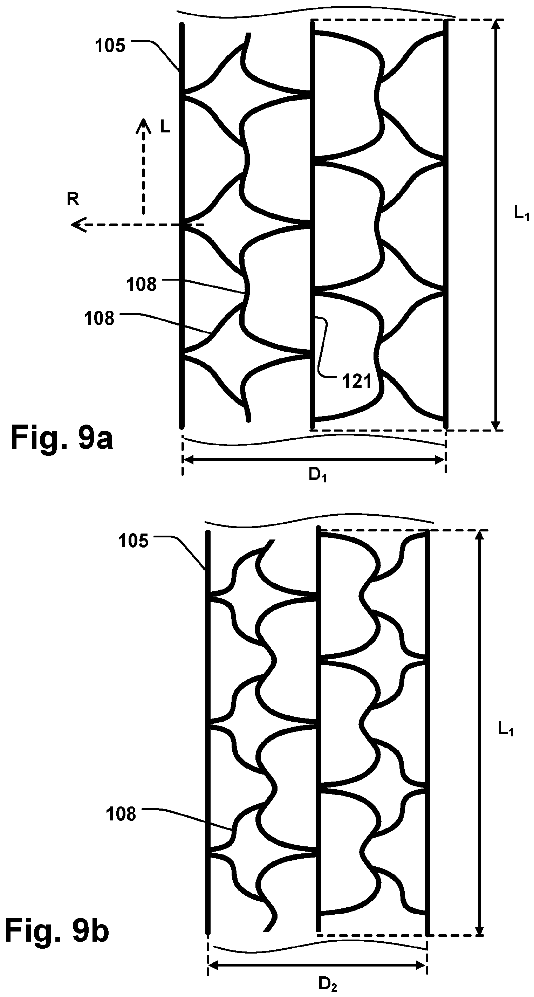

The stent 105 may be radially contractible along a radial direction (R), perpendicular to a longitudinal direction (L) of the stent 105, so that the stent 105 exerts a force (F) on the first and/or second support ring 101, 102. The radial (R) and longitudinal direction (L) of the stent 105 is schematically indicated in Fig. 3. The examples illustrated in Figs. 5a-b are cross-sectional views of the annuloplasty device 100, illustrating the stent 105 arranged around the first- or second support ring 101, 102, and a retention unit 104 in the expanded state (p₂). The stent 105 may thus be radially contractible towards the first and/or second support ring 101, 102, thereby exerting a force (F) onto the latter as indicated with arrows F in the illustrations of Figs. 5a-b. The stent 105 may thus assume a fixed position in relation to the first and/or second support ring 101, 102, as the force (F) creates friction between the stent 105 and the first and/or second support ring 101, 102. The framework of the stent 105 may thus be cut to allow movement in the radial direction (R), i.e. allowing the support elements 108 of the framework to move in relation to eachother, so that the diameter (D) of the stent 105 is variable, as further described with reference to Figs. 8a-b and 9a-b. The stent 105 may be resiliently expandable in the radial direction (R) so that the stent 105 may be expanded to a radially stretched state. The stent 105 may then strive towards a contracted relaxed state with an inner diameter being less than the inner diameter in the radially stretched state. The inner diameter in the radially stretched state may be denoted dₛ (see e.g. Fig. 5a-b) and may be more or equal to an outer diameter (dᵣ) of first and/or second support ring 101, 102. The stent 105 may thus be positioned over the first and/or second support ring 101, 102, when in the radially stretched state. The stent 105 will thus strive towards the contracted relaxed state with a reduced inner diameter, and accordingly exert the aforementioned force (F) on the first and/or second support ring 101, 102. This provides for a facilitated fixation of the position of the stent 105 in relation to the first and/or second support ring 101, 102. The example in Fig. 5a shows a cover 106 between the stent 105 and the first and/or second support ring 101, 102, as described in more detail below. The difference between dᵣ and dₛ is thus larger in this case.

The stent 105 may comprise a shape-memory material in one example. Activation of the shape-memory material may cause the stent 105 to contract to a reduced diameter, along the radial direction R, to apply a force (F) on the first and/or second support ring 101, 102. For example, the shape-memory material may be temperature activated, so that the stent 105 strives towards a reduced inner diameter when subject to heating to the body temperature. This provides for increasing the force (F) exerted on the first and/or second support ring 101, 102, to attain a secure fixation of the stent 105 thereto.

Figs. 8a-b and 9a-b show examples of a radially contractible and expandable stent 105. The stent 105 may comprise support elements 108 configured to be contractible and expandable so that an outer diameter (D₁, D₂) of the stent 105 is variable between an expanded diameter (D₁) and a contracted diameter (D₂) while a predefined length (L₁) of the stent 105 is essentially maintained. Thus, as the support elements 108 undergo a movement and the diameter varies between D₁ and D₂, the overall length (L₁) of the stent 105 may be fixed. Figs. 8a-b and 9a-b show a length L₁ of a section of the stent 105 but it should be understood that this may correspond to the overall or total length of the stent 105 in the longitudinal direction (L). The stent 105 thus exhibit limited or no contraction in the longitudinal direction (L) as the diameter (D₁, D₂) varies. The stent 105 may be expanded to D₁ and positioned on the first and/or second support ring 101, 102, and subsequently retracted to D₂ to apply a force radially inwards and thereby fix its position on the first and/or second support ring 101, 102, while undergoing no or insignificant variations in the length (L₁) of the stent 105. This provides for a facilitated and more accurate positioning of the stent 105 on the first and/or second support ring 101, 102. The tailoring of the stent 105 to first and second support rings 101, 102, of different dimensions is thus facilitated as the position and coverage of the stent 105 over the first and/or second support ring 101, 102, can be predicted with improved accuracy.

The support elements 108 may have different shapes configured to predominantly allow movement of the overall framework of the stent 105 in the radial direction (R), with limited or no movement in the longitudinal direction (L). Figs. 8a-b show an example where the support elements 108 are curved in S-shapes. The S-shaped support elements 108 are stretched when applying a force in the radial direction (R) so that the stent 105 assumes the expanded diameter (D₁) in Fig. 8a. As the external force is released, the S-shaped support elements 108 may contract again so that the stent 105 may assume the contracted diameter (D₂) in Fig. 8b. The length L₁ of the stent 105 is not changed. It should be understood that some movement may occur that could have minor affect on the length (L₁) of the stent 105, but that the movement in the radial direction (R) is significantly larger than the movement in the longitudinal direction (L). The ratio between the movement in the radial direction (R) and the movement in the longitudinal direction (L) may for example be in the range 5:1 to 10:1 for a particularly advantageous and improved fixation of the stent 105 on the first and/or second support ring 101, 102. It is conceivable that the support elements 108 may be curved in different shapes to allow the radial movement as described, such as C-shapes or Z-shapes.

Figs. 9a-b show an example where the support elements 108 are curved in bow-shapes. The bow-shaped support elements 108, or the S-shaped support elements 108, may be arranged to form an essentially cylindrical shape of the stent 105. The support elements 108 may thus extend generally across a surface of such cylindrical shape. The bow-shaped support elements 108 may be stretched when applying a force in the radial direction (R) so that the stent 105 may assume the expanded diameter (D₁) in Fig. 9a. As the external force is released, the bow-shaped support elements 108 may contract again so that the stent 105 may assume the contracted diameter (D₂) in Fig. 9b. As described above, there is no or insignificant movement of the stent 105 in the longitudinal direction (L) compared to the movement in the radial direction (R).

As exemplified in Figs. 8a-b and 9a-b, the support elements 108 may comprise an elongated main frame 121 extending essentially along the longitudinal direction (L) of the stent 105. The elongated main frame 121 may define the aforementioned predefined length (L₁) of the stent 105. The elongated main frame 121 may thus have an essentially fixed position in the longitudinal direction (L) when the outer diameter of the stent 105 varies between the expanded diameter (D₁) and the contracted diameter (D₂). This provides for effectively controlling the length of the stent 105 in the longitudinal direction (L).

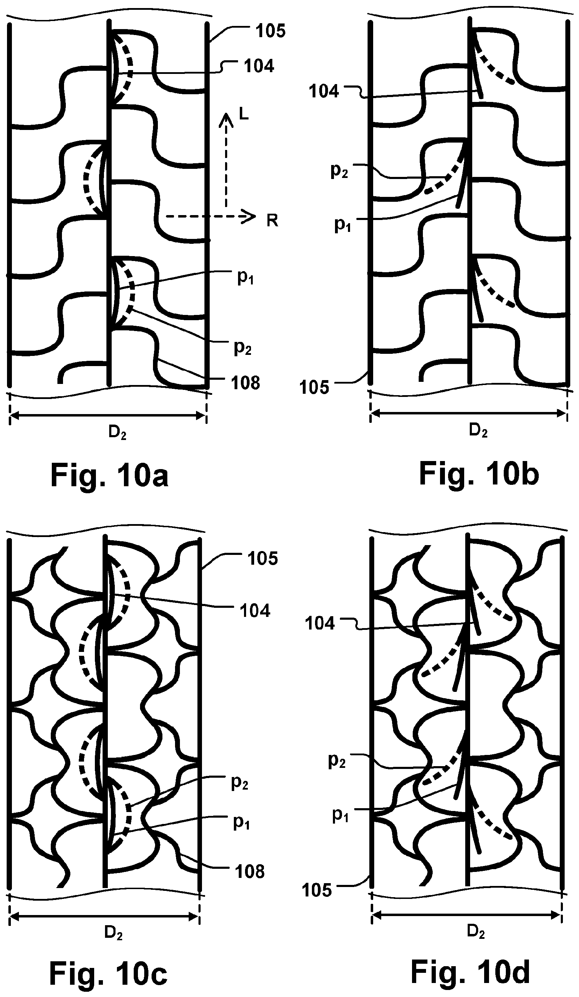

Figs. 10a-d show examples of the stent 105 having different support elements 108 and retention units 104. Figs. 10a-b show an example where the support elements 108 are S-shaped. The retention units 104 may be shaped as expandable bows, as described above, and schematically illustrated in Fig. 10a. A retracted state (p₁) is shown, as well as an expanded state (p₂) where the bows are illustrated with dashed lines. The retention unit 104 may be part of the support elements 108 as described in relation to Figs. 4d-e. As mentioned, the support elements 108 may comprise an elongated main frame 121. In the latter case, the retention unit 104 may be part of the elongated main frame 121. Fig. 10b show an example where the retention units 104 may be shaped as expandable prongs or hook-like structures. A retracted state (p₁) is shown, as well as an expanded state (p₂) where the prongs or hook-like structures are illustrated with dashed lines. Figs. 10c-d illustrate further examples where the bow-like (Figs. 10c) or hook-like (Fig. 10d) retention units 104 are arranged on bow-like support elements 108 of a stent 105. A retracted state (p₁) is shown, as well as an expanded state (p₂). The retention units 104 may be movable between the retracted state (p₁) and the expanded state (p₂) when the stent 105 has the contracted outer diameter (D₂), e.g. when fixed to the first and/or second support ring 101, 102, in order to anchor the stent 105 into the valve tissue.

The annuloplasty device 100 may comprise a cover 106 arranged around at least a portion of the first and/or second support ring 101, 102. The cover 106 may be configured to promote endothelialization and the ingrowth of cells over the annuloplasty device 100. For example, the cover 106 may have a surface which is more porous than the surface of the first- and second support rings 101, 102, which promotes the growth of cells over the annuloplasty device 100. The cover 106 may comprise a weave of a textile or a polymer. The stent 105 may be arranged around at least a portion of the cover 106. The cover 106 may be arranged around the entire length of the first- and second support rings 101, 102.

The stent 105 may exert a force onto the cover 106 so that the cover 106 is pinched between the stent 105 and the first and/or second support ring 101, 102, as exemplified in the schematic illustration of Fig. 5a. Having a cover 106 pinched between the stent 105 and the first and/or second support ring 101, 102, provides for attaining a secure fixation of the position of the cover 106 and the stent 105 relative the first and/or second support ring 101, 102. The stent 105 may thus strive towards an inner diameter which is smaller than an outer diameter of the cover 106 when the latter is arranged around the first and/or second support ring 101, 102, so that a force (F) is exerted radially inwards and pinches the cover 106 against the outer surface of the first and/or second support ring 101, 102. In case the stent 105 is formed from a temperature activated shape-memory material, the stent 105 may increase the force (F) radially inwards as the stent 105 is heated to the body temperature, which further increases the strength of the fixation of the stent 105 relative the first and/or second support ring 101, 102.

The first support ring 101 may comprise a first posterior bow 113 and a first anterior portion 114. The second support ring 101 may comprise a second posterior bow 113' and a second anterior portion 114'. The first and second posterior bows 113, 113', may be adapted to conform to a posterior aspect of the heart valve, i.e. along the posterior leaflet, having a bow-shaped extension. The first and second anterior portions 114, 114', may each have a straighter extension or at least an extension which is less bent than the bow-shaped posterior sides 113, 113'. This is exemplified in Fig. 7b. The first and second anterior portions 114, 114', may thus be adapted to conform to an anterior aspect of the heart valve, i.e. along an anterior leaflet. As mentioned, the first support ring 101 may be adapted to be arranged on an atrial side of the heart valve, and the second support ring 102 may be adapted to be arranged on a ventricular side of the heart valve, as exemplified in Fig. 7a. Fig. 7b show schematic top-down view where the second ring 102 is shown with dashed lines and the first ring 101 is shown with a solid line. The transition point between the first and second rings 101, 102, is in the example of Fig. 7b at the commissure denoted 302'.

The first anterior portion 114 may comprises an anterior stent 105c. The anterior stent 105c comprises a plurality of retention units 104 extending towards the second support ring 102 in their expanded state (p₂), as schematically illustrated in Fig. 1. The second anterior portion 114' may comprise a smooth surface free from retention units 104, as further shown in the example of Fig. 1. This provides for a secure anchoring into the tissue with the first anterior portion 114 at the atrial side, while at the same time the risk of tissue damage is minimized in the ventricle along the second anterior portion 114'.

The first posterior bow 113 may comprise a first posterior stent 105a. The first posterior stent 105a may comprise a first plurality of retention units 104 extending towards the second support ring 102 in their expanded state (p₂), as schematically illustrated in Fig. 1. The second posterior bow 113' may comprise a second posterior stent 105b. The second posterior stent 105b may comprise a second plurality of retention units 104' extending in a direction towards the first plurality of retention units 104. The first and second pluralities of retention units 104, 104', may thus extend in opposite directions along the axial direction 103. Having retention units 104, 104', at both sides along the first and second posterior bows 113, 113', provides for increasing the retention force and the strength by which the annuloplasty device 100 is fixated at the valve. The retention units 104, 104', engage the tissue from both sides of the heart valve, creating a strong retention force in the radial direction, i.e. perpendicular to the axial direction 103. The first and second supports 101, 102, pinch the tissue from both sides of the valve, so that the retention units 104, 104', a forced into the tissue. The retention units 104, 104', provides for shaping the annulus as desired even with a reduced pinching force, since the retention units 104, 104', provides for fixating the shape of the annulus in the radial direction because of the mentioned retention force. This provides for a more reliable implantation at the heart valve, both in the short term and in the long term.

Each individual retention unit 104, 104', may engage or pierce into the tissue with a short distance, for a minimum amount of injury to the tissue. The sum of the retention force and friction created from all the retention units 104, 104', still provides for a strong fixation into the tissue. The scar healing will be quick since each individual retention unit 104, 104', as relatively small dimensions. This provides for a non-traumatic and still secure fixation of the annuloplasty device 100. Hence, the retention units 104, 104', may provide for tissue fixation at multiple points across the annuloplasty device 100 resulting in reduced forces per fixation point, and no need for bulky stitching device or knotting device. There is further no risk of coronary artery occlusion or coronary sinus perforation. Hence, the annuloplasty device 100 provides for ease of operation, and a less time consuming procedure than stitching.

Having a plurality of separate stents 105a, 105b, 105c, arranged along the posterior bows 113, 113', and the first anterior portion 114 allows further for having the stents 105a, 105b, 105c, displaced from intermediate portions 109 extending therebetween. The intermediate portions 109 as indicated in Fig. 1 have a greater radius of curvature than the posterior bows 113, 113', and the anterior portions 114, 114'. Having stents 105a, 105b, 105c, arranged at a distance from the intermediate portions 109 provides for maintaining a greater flexibility of the first and second support rings 101, 102, along the intermediate portions 109, and a facilitated bending along the latter.

The retention units 104, 104', may be evenly spaced along the stents 105, 105a, 105b, 105c, as exemplified in Fig. 1 and Fig. 2. Such even distribution of the fixation points provides for a reliable anchoring to the tissue, minimizing the risk of localized pressure peaks. It should be understood however that the distance between each of the retention units 104, 104', may be varied to optimize the anchoring annuloplasty device 100 to different anatomies. The first and/or second anterior portion 114, 114', may have 5 to 6 retention units 104, 104', respectively. The first and/or second posterior portion 113, 113', may have 6 to 8 retention units 104, 104', respectively. This may provide for a particularly efficient fixation to the tissue while minimizing the overall tissue penetration. It should be understood however that the number of retention units 104, 104', may be varied to optimize the anchoring annuloplasty device 100 to different anatomies and valves of different size. In one example the length of the retention units 104, 104', is in the range 0.5 - 1.5 mm. In another example the length of the retention units 104, 104', is in the range 0.8 - 1.2 mm, such as 1.0 mm, which may provide for a particularly advantageous fixation into the tissue while being easy to deploy via a delivery catheter.

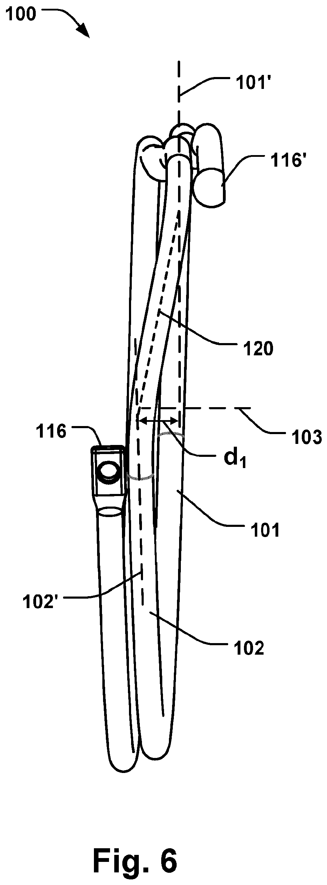

The first support ring 101 may transition to the second support ring 102 over a transition section 120, as illustrated in Fig. 6. The stent 105 has been omitted from Fig. 6 for a clearer illustration. The transition section 120 is adapted to be arranged at a commissure 302, 302', of the heart valve leaflets, e.g. at a commissure 302' as illustrated in Fig. 7b. The first and second support rings 101, 102, extend in respective first and second coil planes 101', 102', being essentially perpendicular to the central axis 103. The transition region 120 may bend at least partly along the central axis 103 so that the first coil plane 101' is separated a distance (d₁) from the second coil plane 102' along the central axis 102 (i.e. along a direction parallel to the central axis) at the transition region 120. Having such transition section 120 where the coil planes 101', 102', are locally displaced a distance (d₁), and at a position corresponding to the location of the commissure 302, 302', provides for improved accommodation of the first and second support rings 101, 102, to the anatomy at the opposite sides of the valve, in particular as the heart beats. This allows for the retention units 104, 104', of the stent 105, 105a, 105b, 105c, to effectively pierce into the tissue as the first and second support rings 101, 102, accommodate to the anatomy.

Further, having a step-down in the coil planes 101', 102', or an "S-shape", or "Z-shape", at the transition region 120 due to separation distance (d₁) provides for a better coaptation of the first and second support rings 101, 102, at the commissure 302, 302'. I.e. the risk of having the moving valve leaflets pulling on any of the support rings 101, 102, at the commissure 302, 302', is minimized because the first coil plane 101' of the first support ring 101 on the atrial side transitions to the second coil plane 102' of the second support ring 102 in a shorter distance at the transition region 120 due to the displacement (d₁). This means that the first and second support rings 101, 102, may conform better to the two opposite sides of the valve close to the commissure 302, 302'. The annuloplasty device 100 may thus be secured at the valve in a safer manner, while the risk of dislocations is minimized. The position of the transition section 120 may be varied depending on which commissure 302, 302', the first/second support rings 101, 102, extend through the valve leaflets. The transition section 120 may thus have an increased slope or pitch relative the central axis 103 compared to the remaining portions of the first and second support rings 101, 102.

The transition section 120 may bend at least partly along a radial direction (r) of the coiled configuration of the first and second support rings 101, 102, where the radial direction (r) is perpendicular to the central axis 103, so that the transition section 120 is concave towards the radial direction (r). Such concave bend, or "C-curve", of the transition section 120 towards the radial direction (R) provides for further improving the coaptation of the first and second support rings 101, 102, to the valve anatomy close to the commissure 302, 302'. The risk of having a disadvantageous force transfer or friction between the moving valve leaflets and any of the support rings 101, 102, at the commissure 302, 302', is minimized. The first and second support rings 101, 102, may extend along the annulus as far as possible while extending through the commissure 302, 302', with minimized impact on the valve motion, as the concave bend of the transition section 120 allows for adapting to anatomies where the commissure 302, 302', is located loser to the central axis 103 than the annulus. The annuloplasty device 100 may thus be secured at the valve in a further improved manner, while the risk of dislocations in the long term is minimized.

The first and second support rings 101, 102, may have respective free ends 116, 116', as illustrated in Fig. 1. The free ends 116, 116', may be configured to be arranged on opposite sides of the native heart valve leaflets. The two free ends 116, 116', may be displaced from each other with a peripheral off-set distance extending in a coil plane. The coil plane is substantially parallel to an annular periphery of the coil formed by the first and second support rings 101, 102, and perpendicular to the axial direction 103. The coil plane accordingly corresponds to the plane spanned by the annular periphery of the device 100 when in the coiled configuration. The peripheral off-set distance between the two free ends 116, 116', thus extends substantially perpendicular to the central axis 103. This means that, when the device 100 is positioned in the implanted state, around the annulus of the heart valve, the two free ends 116, 116', will be separated along the plane of the valve. Having such off-set 117 in the plane of the valve, resulting in a reduced length of the first or second support rings 101, 102, may be advantageous in some anatomies where there might be a risk of interference of the first or second support rings 101, 102, with the valve motion.

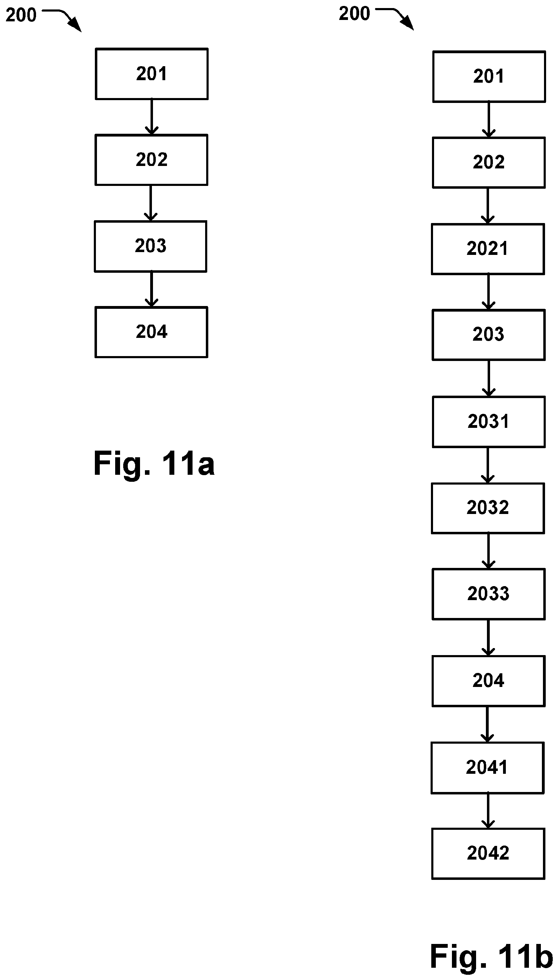

A method 200 of repairing a defective heart valve is disclosed. The method 200 is schematically illustrated in Fig. 11a, in conjunction with Figs. 1 - 10, 12 - 16. The order in which the steps are described should not be construed as limiting, and it is conceivable that the order of the steps may be varied depending on the particular procedure. The method 200 comprises positioning 201 a first support ring 101 of an annuloplasty device 100 on a ventricular side of the heart valve, and positioning 202 a second support ring 102 of the annuloplasty device on an atrial side of the heart valve. The first and second support rings are thus arranged as a coil extending through a commissure 302, 302' of the heart valve. The first and/or second support ring 101, 102, comprises a stent 105, 105a, 105b, 105c arranged around at least a portion of the first and/or second support ring 101, 102. The stent comprises retention units 104, 104'. The method 200 comprises positioning 203 the stent 105 in abutment with valve tissue along the aforementioned portion of the first and/or second support ring 101, 102, so that the retention units 104, 104', are engaged 204 into tissue of the heart valve. The method 200 provides for the advantageous benefits as discussed in relation to the annuloplasty device 100 and Figs. 1 - 10, 12 - 16. The method 200 allows for a facilitated anchoring of the annuloplasty device 100 at the heart valve, due to the robust and reliable fixation mechanism provided by stents 105, 105a, 10b, 105c, and the retention units 104, 104', fixed thereto.

A further method 200 is schematically illustrated in Fig. 11b. The method 200 may comprise positioning 2031 an anterior stent 105c on the atrial side along a first anterior portion 114 of the first support ring 101, and positioning 2032 a first posterior stent 105a on the atrial side along a first posterior bow 113 of the first support ring 101. The method 200 may further comprise positioning 2033 a second posterior stent 105b on the ventricular side along a second posterior bow 113' of the second support ring 102.

The method 200 may further comprise engaging 2041 retention units 104 of the anterior stent 105c into valve tissue on the atrial side, and engaging 2042 retention units 104, 104', of the first and second posterior stents 105a, 105b, into valve tissue on the respective atrial and ventricular side to anchor the annuloplasty device 100 at the heart valve. A secure fixation of the annuloplasty device at the opposite sides of the heart valve leaflets is thus provided, as described with respect to Figs. 1 - 10, 12 - 16.

The method 200 may comprise positioning the first and second support rings 101, 102, on the respective atrial and ventricular sides of the heart valve by ejecting 2021 the first and second support rings from a delivery catheter (not shown), where the retention units 104, 104', move from a retracted state (p₁) to an expanded state (p₂) as the first and second support rings 101, 102, are ejected from the delivery catheter.

The retention units 104, 104', may be aligned essentially flush with an outer diameter (D) of the stent 105, 105a, 105b, 105c, in the retracted state (p₁) when the first and second support rings 101, 102, moves along a lumen if the delivery catheter.

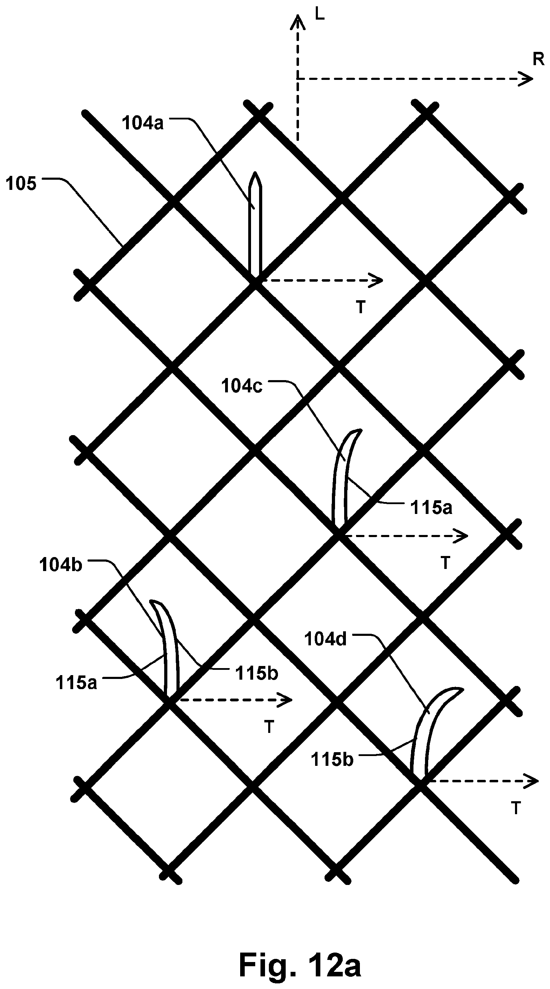

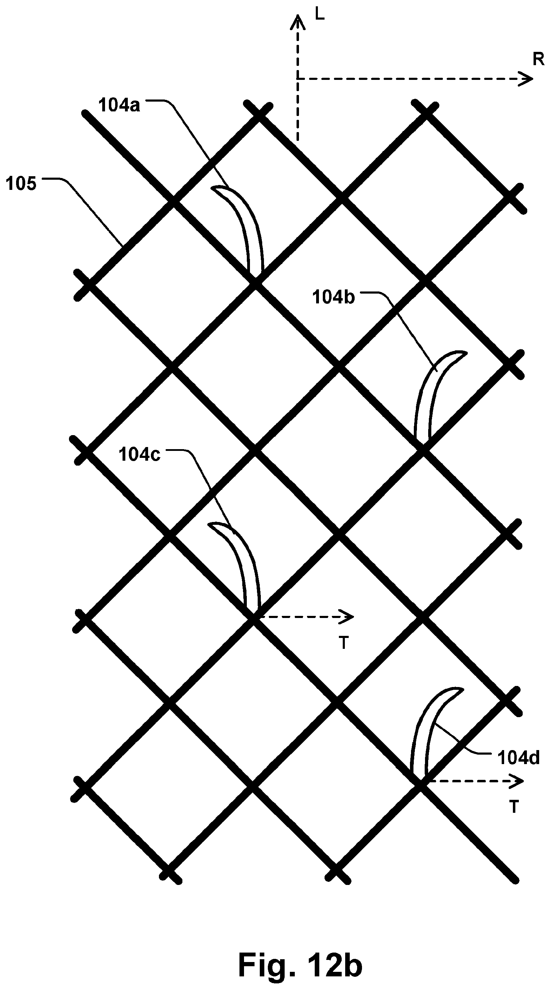

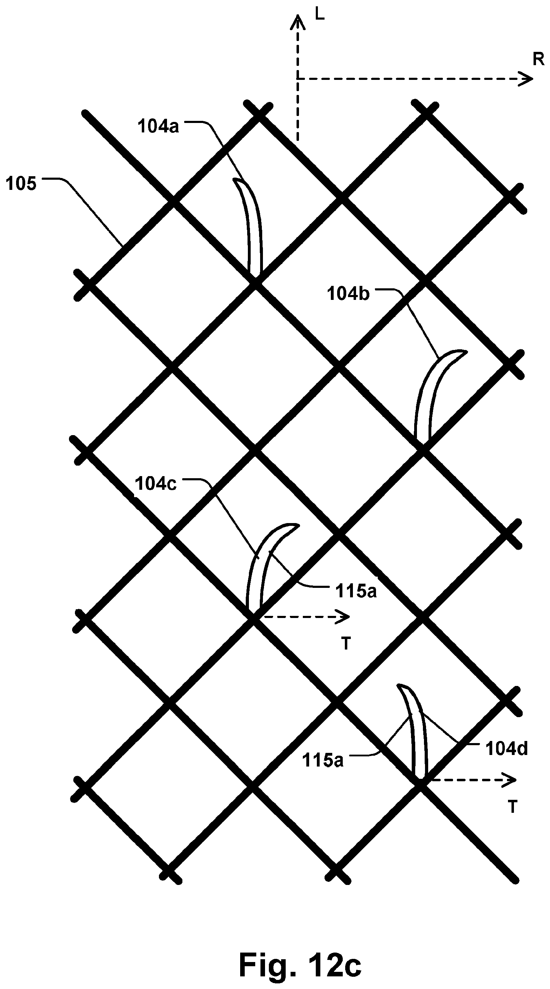

The retention units 104, 104', may be curved in some examples. Figs. 12 - 13, 15 - 16 are schematic illustrations of retention units 104a-d having curved shapes. Any of the stents 105, 105a, 105b, 105c, as described above may have curved retention units 104a-d. The stent will be denoted with reference numeral 105 below for brevity. Retention units 104, 104', as described above may be retention units 104a-d as described in relation to Figs. 12 - 16.

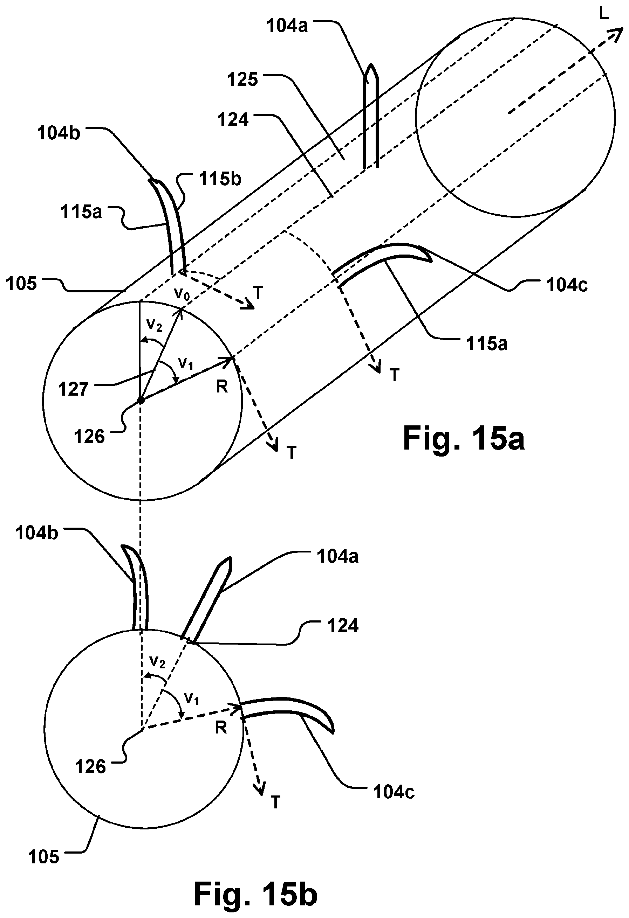

The retention units 104a-d may comprise an at least partly concave surface 115a, or an at least partly convex surface 115b, towards the direction of a respective tangent vector (T) at the respective positions of the retention units 104a-d on the stent 105. The tangent vector (T) is perpendicular to a radial direction (R) of the stent 105 as well as to a longitudinal direction (L) of the stent 105. The radial direction (R) is perpendicular to the longitudinal direction (L). Fig. 15a shows an example of the stent 105 in a perspective view where a retention unit 104c is curved by having a concave shape towards the tangent vector (T) at the radial position of the retention unit 104c on the circumferential surface 125 of the stent 105. The radial position is in this example indicated by the angle v₁ relative a longitudinal reference line 124 on the surface 125. The surface 125 may be construed as a tubular shape with a radius corresponding to the radius of the stent framework, as schematically indicated by the retention unit 104c extending from the surface 125. The longitudinal reference line 124 may be defined as a reference position on the stent 105, and may be positioned in a direction 127 from a center 126 of the stent 105, where the direction 127 represents a reference "zero" angle v₀ from the center 126, as indicated in the example of Fig. 15a. The direction 127 may be defined to be aligned with other reference directions, for example aligned in parallel with central axis 103 (Fig. 1).

Fig. 15 shows an example of a retention unit 104b being curved with a convex surface 115b facing the direction of the associated tangent vector (T) on the surface 125 where the retention unit 104b is positioned, indicated at radial position v₂, relative the "zero" angle v₀. The tangent vectors (T) of the respective retention units 104a-d, are oriented in the same clockwise direction throughout the examples, provided the circumferential cross-section of the stent 105 (Fig. 15b) as viewed from the indicated center point 126 and along the longitudinal direction (L) in Fig 15a. Fig. 12a shows a section of the stent 105 and a corresponding alignment of retention units 104b and 104c as exemplified in Figs. 15a-b. I.e. the retention units 104b, 104c, have convex and concave surfaces 115b, 115a, respectively, towards the tangent vector (T). The example in Figs. 12a and 15a shows retention unit 104a extending essentially straight so that there is no curvature in the direction of the tangent vector (T). It should be understood that the stent 105 may comprise any combination of retention units 104a-d having different curvatures or extending straight. Further examples are described with reference to Figs. 12a-c, 13, 15-16.

Having curved retention units 104a-d as described above provides for an improved grip of the stent 105 into the tissue, and thereby a more reliable fixation of the annuloplasty device 100. The first and second support rings 101, 102, are subject to the repeated movement pattern of the beating heart, when placed at the opposite sides of the heart valve. Curved retention units 104a-d provide for an advantageous directionality, as well as variability, of the retention force by which the respective unit 104a-d is anchored into the tissue. The risk for migration and dislocation over time of the first and second support rings 101, 102, onto which the retention units 104a-d are arranged, can thus be reduced. Having retention units 104a-d which are curved in different directions as exemplified above with reference to Figs. 12a and 15a-b provides for an advantageous anchoring into the valve tissue with such reduced risk of migration of the annuloplasty device 100. For example, the curvature of retention unit 104c in Fig, 15b generates a retention force with a force component aligned in the direction of v₁ towards the center 126, while curved retention unit 104b generates a retention force with a force component aligned in the direction of v₂ towards the center 126. The two aforementioned force components thus intersect with an angle (v₁ + v₂) at the center 126 which provides for a secure fixation of the position of the stent 105, and thereby the first and/or second support ring 101, 102, onto which the stent 105 is arranged.

The retention units 104a-d may be curved in opposite directions so that a concave surface 115a of at least a first retention unit 104b faces the opposite direction of a concave surface 115a of at least a second retention unit 104c. Fig. 12a shows an example of a first retention unit 104b being curved opposite a second retention unit 104c. The associated tangent vectors (T) are directed in the same clockwise direction, as described above in relation to Fig. 15a. The concave surface 115a of the first retention unit 104b face away from the direction of the tangent vector (T), while the concave surface 115a of the second retention unit 104c face the same direction as the tangent vector (T). Fig. 12c shows another example where retention units 104c and 104d are curved in opposite directions. The stent 105 may have retention units 104a-d being curved in any combination of directions. Having retention units 104a-d curved in opposite directions provides further for efficiently diverting the retention force with a greater angular spread, and thereby for reducing the risk of dislocation of the stent 105 and for an efficient anchoring into the tissue.

The retention units 104a-d may be curve towards eachother. **In** the example of Fig. 12c, the first retention unit 104c is curved towards the second retention unit 104d. **In** another example the first retention unit 104c and the second retention unit 104d are curved away from eachother, as schematically illustrated in Fig. 12b. The stent 105 may have retention units 104a-d being curved in away from eachother, or towards eachother, in any combination.

The amount of curvature of the curved shape may vary with an angle (v₁, v₂) at which the respective retention units 104a-d are positioned around a circumference of the stent 105. Figs. 15a-b show an example where retention unit 104c arranged at angle v₁, relative v₀, has a curved surface 115a which is different from the curved surface 115a of retention unit 104b arranged at v₂. The radius of curvature of the aforementioned curved surfaces 115a may be different. E.g. the radius of curvature of retention unit 104c may be greater than the radius of curvature of retention unit 104b. The example in Fig. 12b shows a further retention unit 104a having the least radius of curvature, e.g. an essentially straight extension. Fig. 12a and 12c show further examples where the radius of curvature of the retention units 104a-d varies with their respective positions around the circumference of the stent 105. This provides for optimizing the retention force of the retention units 104a-d in dependence on which part on the first and/or second support ring 101, 102, the retention units 104a-d are placed. The first and second support rings 101, 102, are placed at the heart valve where the surrounding anatomy varies along the different parts of the first and second support rings 101, 102. The placement and curvatures of the retention units 104a-d may thus be optimized to such varying anatomy to further increase the reliability of the fixation.

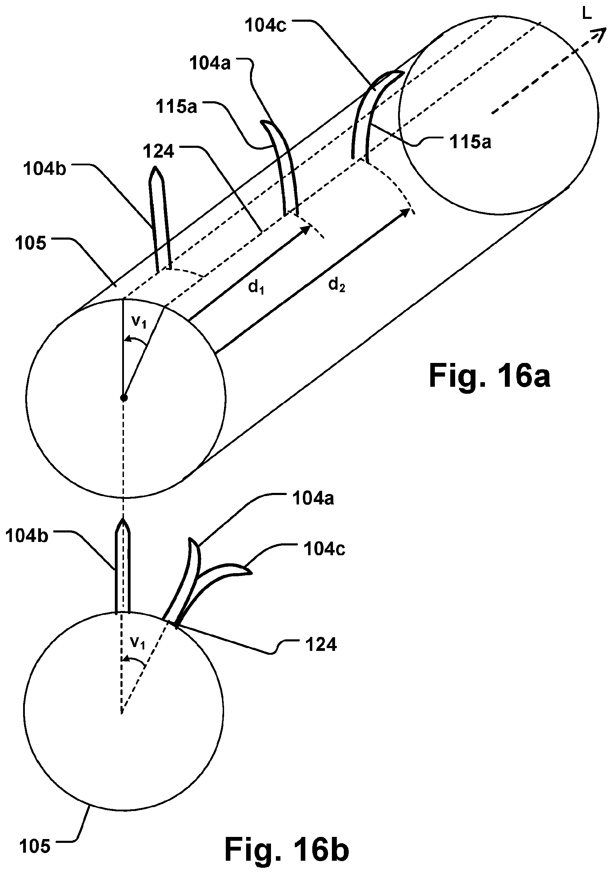

The amount of curvature of the curved shape may vary with a position (d₁, d₂) along the longitudinal direction (L) of the stent 105 at which the respective retention unit 104a-d is positioned. Figs. 16a-b show an example where retention unit 104a arranged at position d₁, along longitudinal direction (L), has a curved surface 115a which is different from the curved surface 115a of retention unit 104c arranged at a different position d₂ along the longitudinal direction (L). The radius of curvature of the aforementioned curved surfaces 115a may be different. E.g. the radius of curvature of retention unit 104c may be greater than the radius of curvature of retention unit 104a. Fig. 12c show a further example where the radius of curvature of the retention units 104a-d varies with their respective positions along the longitudinal direction (L) of the stent 105. This provides for optimizing the retention force of the retention units 104a-d in dependence on which part on the first and/or second support ring 101, 102, the retention units 104a-d are placed. The first and second support rings 101, 102, are placed at the heart valve where the surrounding anatomy varies along the different parts of the first and second support rings 101, 102. The placement and curvatures of the retention units 104a-d may thus be optimized to such varying anatomy to further increase the reliability of the fixation.

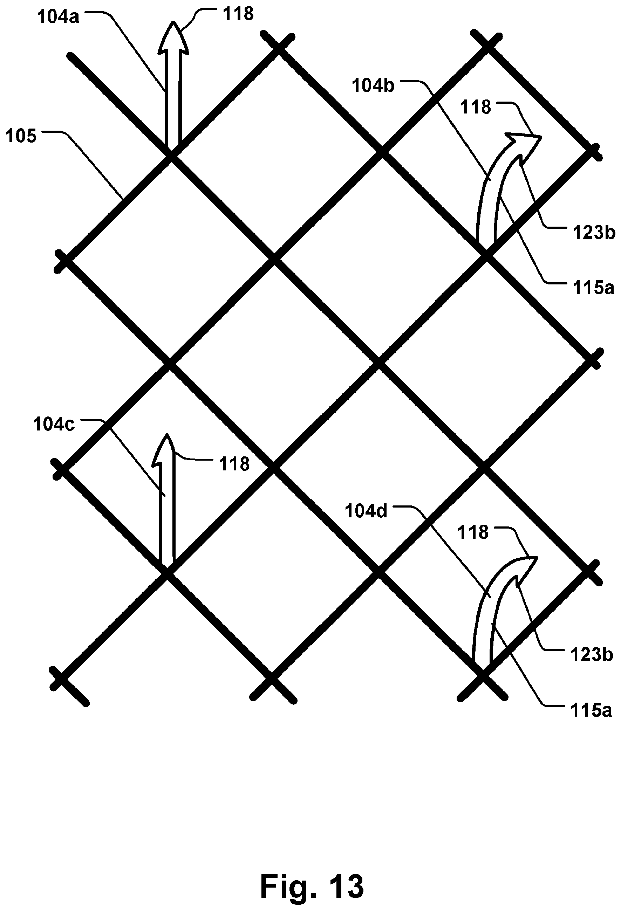

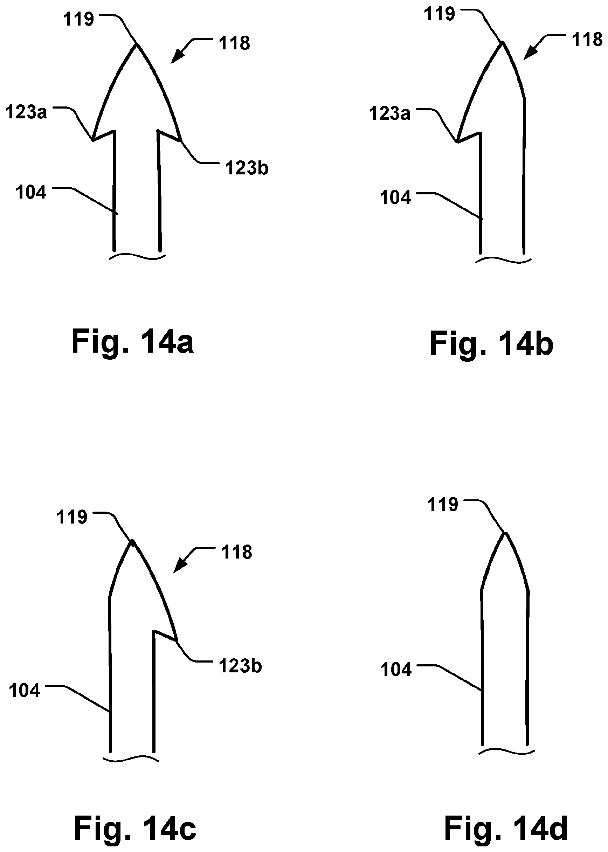

The retention units 104a-d may have a serrated or hook shaped spike 118, as schematically illustrated in Figs. 13 and 14a-c. This provides for further optimizing the retention force into the tissue. Fig. 14a shows an example where the retention unit 104 tapers to a tip 119 and having an arrow shape with hooks 123a-b at either side of the tip 119. Fig. 14b shows a retention unit 104 with a tip 119 and a single hook 123a at one side of the tip 119. Fig. 14c shows an example where the hook 123b is positioned at the opposite side of the tip 119, compared to Fig. 14b, and Fig. 14d shows a retention unit 104 tapering to a single tip 119 without hooks. Any of the curved retention units 10a-d as described above may have a serrated or hook shaped spike 118. Fig. 13 shows an example where retention units 104a-d have hook-shaped spikes 118. Retention units 104b and 104d are curved whereas retention units 104a and 104c are essentially straight. **In** one example the hook 123b is provided at the concave surface 115a of the retention unit 104a-d, e.g. as illustrated for retention units 104b and 104d in Fig. 13. This may provide for an increased retention force into the tissue when combined with the curvature of the concave surface 115a.

The present invention has been described above with reference to specific embodiments. However, other embodiments than the above described are equally possible within the scope of the invention. The different features and steps of the invention may be combined in other combinations than those described. The scope of the invention is only limited by the appended patent claims. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings of the present invention is/are used.

## Claims

1. An annuloplasty device (100) comprising
first (101) and second (102) support rings having a coiled configuration in which the first and second support rings are arranged as a coil around a central axis (103),
wherein the first and second support rings are configured to be arranged on opposite sides of native heart valve leaflets (301) of a heart valve,
a stent (105, 105a, 105b, 105c) arranged around at least a portion of the first and/or second support ring, and
wherein the stent comprises retention units (104, 104', 104a, 104b, 104c, 104d), wherein
the retention units have a curved shape comprising an at least partly concave or convex (115a, 115b) surface towards the direction of a respective tangent vector (T) at the respective positions of the retention units on the stent, the tangent vector is perpendicular to a radial direction (R) of the stent and a longitudinal direction (L) of the stent, and the radial direction is perpendicular to the longitudinal direction, and
wherein the retention units are curved in opposite directions so that a convex surface of at least a first retention unit (104b) faces the opposite direction of a convex surface of at least a second retention unit (104d).

2. Annuloplasty device according to claim 1, wherein the retention units are formed from the material of the stent, whereby the retention units are integrated with the stent.

3. Annuloplasty device according to any of claims 1 - 2, wherein the retention units are resiliently moveable from a retracted state (p₁) to an expanded state (p₂), and wherein the retention units are flexible to bend from the expanded state (p₂) to the retracted state (p₁).

4. Annuloplasty device according to claim 3, wherein the retention units are aligned essentially flush with an outer diameter (D) of the stent in the retracted state.

5. Annuloplasty device according to any of claims 1 - 4, wherein the stent is radially contractible along a radial direction (R), perpendicular to a longitudinal direction (L) of the stent, so that the stent exerts a force (F) on the first and/or second support ring.

6. Annuloplasty device according to any of claims 1 - 5, wherein the stent comprises support elements (108) configured to be contractible and expandable so that an outer diameter (D₁, D₂) of the stent is variable between an expanded diameter (D₁) and a contracted diameter (D₂) while a predefined length (L₁) of the stent is essentially maintained.

7. Annuloplasty device according to claim 6, wherein the support elements comprise an elongated main frame (121) extending essentially along the longitudinal direction (L) of the stent, wherein the elongated main frame defines the predefined length (L₁) of the stent, whereby the elongated main frame has an essentially fixed position in the longitudinal direction (L) when the outer diameter of the stent varies between the expanded diameter (D₁) and the contracted diameter (D₂).

8. Annuloplasty device according to any of claims 1 - 7, comprising a cover (106) arranged around at least a portion of the first and/or second support ring, and
wherein the stent is arranged around at least a portion of the cover, wherein the stent exerts a force onto the cover so that the cover is pinched between the stent and the first and/or second support ring.

9. Annuloplasty device according to any of claims 1 - 8, wherein the first support ring comprises a first posterior bow (113) and a first anterior portion (114),
the second support ring comprises a second posterior bow (113') and a second anterior portion (114'),
the first and second posterior bows are adapted to conform to a posterior aspect of said heart valve, and the first and second anterior portions are adapted to conform to an anterior aspect of said heart valve,
the first anterior portion (114) comprises an anterior stent (105c), the anterior stent comprising a plurality of retention units, and
the second anterior portion (114') comprises a smooth surface free from retention units.

10. Annuloplasty device according to claim 9, wherein the first posterior bow (113) comprises a first posterior stent (105a) comprising a first plurality of retention units, and
the second posterior bow (113') comprises a second posterior stent (105b) comprising a second plurality of retention units extending in a direction towards the first plurality of retention units.

11. Annuloplasty device according to claim 1, wherein the first retention unit is curved towards the second retention unit.

12. Annuloplasty device according to claim 1, wherein the first and second retention units are curved away from eachother.

13. Annuloplasty device according to any of claims 1 - 12, wherein the amount of curvature of the curved shape varies with an angle (v₁, v₂) at which the respective retention units are positioned around a circumference of the stent.

14. Annuloplasty device according to any of claims 1 - 13, wherein the amount of curvature of the curved shape varies with a position (d₁, d₂) along the longitudinal direction (L) of the stent at which the respective retention unit is positioned.

15. Annuloplasty device according to any of claims 1 - 14, wherein the retention units have a serrated or hook shaped spike (118).

## Patentansprüche

1. Anuloplastievorrichtung (100), umfassend:
einen ersten (101) und einen zweiten (102) Stützring mit einer gewundenen Auslegung, in der der
erste und der zweite Stützring als eine Windung um eine Mittelachse (103) angeordnet sind,
wobei der erste und der zweite Stützring dazu ausgelegt sind, auf gegenüberliegenden Seiten der natürlichen Herzklappensegel (301) einer Herzklappe angeordnet zu werden,
einen Stent (105, 105a, 105b, 105c), der um mindestens einen Abschnitt des ersten und/oder des zweiten Stützrings herum angeordnet ist, und
wobei der Stent Halteeinheiten (104, 104', 104a, 104b, 104c, 104d) umfasst, wobei die Halteeinheiten eine gekrümmte Form haben und eine mindestens teilweise konkave oder konvexe (115a, 115b) Oberfläche in Richtung der Richtung eines jeweiligen Tangentenvektor (T) in den jeweiligen Positionen der Halteeinheiten an dem Stent umfassen, wobei der Tangentenvektor senkrecht zu einer radialen Richtung (R) des Stents und einer Längsrichtung (L) des Stents ist, und die radiale Richtung senkrecht zu der Längsrichtung ist, und
wobei die Halteeinheiten in entgegengesetzte Richtungen gebogen sind, so dass eine konvexe Oberfläche von mindestens einer ersten Halteeinheit (104b) der entgegengesetzten Richtung einer konvexen Oberfläche von mindestens einer zweiten Halteeinheit (104d) zugewandt ist.

2. Anuloplastievorrichtung nach Anspruch 1, wobei die Halteeinheiten aus dem Material des Stents gebildet sind, wodurch die Halteeinheiten in den Stent integriert sind.

3. Anuloplastievorrichtung nach einem der Ansprüche 1 - 2, wobei die Halteeinheiten federnd aus einem zurückgezogenen Zustand (p₁) in einen expandierten Zustand (p₂) bewegbar sind, und wobei die Halteeinheiten flexibel sind, um sich aus dem expandierten Zustand (p₂) in den zurückgezogenen Zustand (p₁) zu biegen.

4. Anuloplastievorrichtung nach Anspruch 3, wobei die Halteeinheiten in dem zurückgezogenen Zustand im Wesentlichen bündig auf einen Außendurchmesser (D) des Stents ausgerichtet sind.

5. Anuloplastievorrichtung nach einem der Ansprüche 1 - 4, wobei der Stent entlang einer radialen Richtung (R), senkrecht zu einer Längsrichtung (L) des Stents, radial kontrahierbar ist, so dass der Stent eine Kraft (F) auf den ersten und/oder den zweiten Stützring ausübt.

6. Anuloplastievorrichtung nach einem der Ansprüche 1 - 5, wobei der Stent Stützelemente (108) umfasst, die dazu ausgelegt sind, kontrahierbar und expandierbar zu sein, so dass ein Außendurchmesser (D₁, D₂) des Stents zwischen einem expandierten Durchmesser (D₁) und einem kontrahierten Durchmesser (D₂) variabel ist, während eine vordefinierte Länge (L₁) im Wesentlichen beibehalten wird.

7. Anuloplastievorrichtung nach Anspruch 6, wobei die Stützelemente ein längliches Hauptgerüst (121) umfassen, das sich im Wesentlichen entlang der Längsrichtung (L) des Stents erstreckt, wobei das längliche Hauptgerüst die vordefinierte Länge (L₁) des Stents definiert, wodurch das längliche Hauptgerüst eine im Wesentlichen feste Position in der Längsrichtung (L) hat, wenn der Außendurchmesser des Stents zwischen dem expandierten Durchmesser (D₁) und dem kontrahierten Durchmesser (D₂) variiert.

8. Anuloplastievorrichtung nach einem der Ansprüche 1 - 7, umfassend eine Abdeckung (106), die um mindestens einen Abschnitt des ersten und/oder des zweiten Stützrings herum angeordnet ist, und
wobei der Stent um mindestens einen Abschnitt der Abdeckung herum angeordnet ist, wobei der Stent eine Kraft auf die Abdeckung ausübt, so dass die Abdeckung zwischen dem Stent und dem ersten und/oder dem zweiten Stützring eingeklemmt ist.

9. Anuloplastievorrichtung gemäß einem der Ansprüche 1 - 8, wobei der erste Stützring einen ersten posterioren Bogen (113) und einen ersten anterioren Abschnitt (114) umfasst,
der zweite Stützring einen zweiten posterioren Bogen (113') und einen zweiten anterioren Abschnitt (114') umfasst,
der erste und der zweite posteriore Bogen dazu angepasst sind, einem posterioren Aspekt der Herzklappe zu entsprechen, und der erste und der zweite anteriore Abschnitt dazu angepasst sind, einem anterioren Aspekt der Herzklappe zu entsprechen,
der erste anteriore Abschnitt (114) einen anterioren Stent (105c) umfasst, wobei der anteriore Stent eine Vielzahl von Halteeinheiten umfasst, und
der zweite anteriore Abschnitt (114') eine glatte Oberfläche ohne Halteeinheiten umfasst.

10. Anuloplastievorrichtung nach Anspruch 9, wobei der erste posteriore Bogen (113) einen ersten posterioren Stent (105a) umfasst, der eine erste Vielzahl von Halteeinheiten umfasst, und
der zweite posteriore Bogen (113') einen zweiten posterioren Stent (105b) umfasst, der eine zweite Vielzahl von Halteeinheiten umfasst, die sich in eine Richtung in Richtung der ersten Vielzahl von Halteeinheiten erstrecken.

11. Anuloplastievorrichtung nach Anspruch 1, wobei die erste Halteeinheit in Richtung der zweiten Halteeinheit gekrümmt ist.

12. Anuloplastievorrichtung nach Anspruch 1, wobei die erste und die zweite Halteeinheit voneinander weg gekrümmt sind.

13. Anuloplastievorrichtung nach einem der Ansprüche 1 - 12, wobei der Krümmungsgrad der gekrümmten Form mit einem Winkel (v₁, v₂) variiert, in dem die jeweiligen Halteeinheiten rund um einen Umfang des Stents positioniert sind.

14. Anuloplastievorrichtung nach einem der Ansprüche 1 - 13, wobei der Krümmungsgrad der gekrümmten Form mit einer Position (d₁, d₂) entlang der Längsrichtung (L) des Stents variiert, in der die jeweilige Halteeinheit positioniert ist.

15. Anuloplastievorrichtung nach einem der Ansprüche 1 - 14, wobei die Halteeinheiten eine gezackte oder hakenförmige Spitze (118) haben.

## Revendications

1. Dispositif d'annuloplastie (100) comprenant
de premier (101) et deuxième (102) anneaux de support ayant une configuration en spirale dans laquelle les premier et deuxième anneaux de support sont disposés en spirale autour d'un axe central (103),
les premier et deuxième anneaux de support étant conçus pour être disposés sur des côtés opposés de feuillets de valve cardiaque naturels (301) d'une valve cardiaque,
un stent (105, 105a, 105b, 105c) disposé autour d'au moins une partie du premier et/ou du deuxième anneau de support, et
dans lequel le stent comprend des unités de retenue (104, 104', 104a, 104b, 104c, 104d), les unités de retenue ayant une forme incurvée comprenant une surface au moins partiellement concave ou convexe (115a, 115b) dans la direction d'un vecteur tangent respectif (T) aux positions respectives des unités de retenue sur le stent, le vecteur tangent étant perpendiculaire à une direction radiale (R) du stent et à une direction longitudinale (1) du stent, et la direction radiale étant perpendiculaire à la direction longitudinale, et
dans lequel les unités de retenue sont incurvées dans des directions opposées, de sorte qu'une surface convexe d'au moins une première unité de retenue (104b) fait face à la direction opposée d'une surface convexe d'au moins une deuxième unité de retenue (104d).

2. Dispositif d'annuloplastie selon la revendication 1, dans lequel les unités de retenue sont formées à partir du matériau du stent, les unités de retenue étant ainsi intégrées au stent.

3. Dispositif d'annuloplastie selon l'une quelconque des revendications 1 et 2, dans lequel les unités de retenue sont mobiles élastiquement d'un état rétracté (p₁) à un état déployé (p₂), et dans lequel les unités de retenue sont souples pour se plier de l'état déployé (p₂) à l'état rétracté (p₁).

4. Dispositif d'annuloplastie selon la revendication 3, dans lequel les unités de retenue sont alignées sensiblement de niveau avec un diamètre extérieur (D) du stent à l'état rétracté.

5. Dispositif d'annuloplastie selon l'une quelconque des revendications 1 à 4, dans lequel le stent est contractable radialement le long d'une direction radiale (R), perpendiculaire à une direction longitudinale (L) du stent, de sorte que le stent exerce une force (F) sur le premier et/ou le deuxième anneau de support.

6. Dispositif d'annuloplastie selon l'une quelconque des revendications 1 à 5, dans lequel le stent comprend des éléments de support (108) conçus pour être contractables et expansibles, de sorte qu'un diamètre extérieur (D₁, D₂) du stent est variable entre un diamètre expansé (D₁) et un diamètre contracté (D₂), tandis qu'une longueur prédéfinie (L₁) du stent est sensiblement maintenue.

7. Dispositif d'annuloplastie selon la revendication 6, dans lequel les éléments de support comprennent une armature principale allongée (121) s'étendant sensiblement le long de la direction longitudinale (L) du stent, l'armature principale allongée définissant la longueur prédéfinie (L₁) du stent, l'armature principale allongée ayant ainsi une position sensiblement fixe dans la direction longitudinale (L) lorsque le diamètre extérieur du stent varie entre le diamètre expansé (D₁) et le diamètre contracté (D₂).

8. Dispositif d'annuloplastie selon l'une quelconque des revendications 1 à 7, comprenant une enveloppe (106) disposée autour d'au moins une partie du premier et/ou du deuxième anneau de support, et
dans lequel le stent est disposé autour d'au moins une partie de l'enveloppe, le stent exerçant une force sur l'enveloppe, de sorte que l'enveloppe est pincée entre le stent et le premier et/ou le deuxième anneau de support.

9. Dispositif d'annuloplastie selon l'une quelconque des revendications 1 à 8, dans lequel le premier anneau de support comprend un premier arc postérieur (113) et une première portion antérieure (114),
le deuxième anneau de support comprend un deuxième arc postérieur (113') et une deuxième portion antérieure (114'),
les premier et deuxième arcs postérieurs étant adaptés pour épouser la forme d'un contour postérieur de ladite valve cardiaque, et les première et deuxième portions antérieures sont adaptées pour épouser la forme d'un contour antérieur de ladite valve cardiaque,
la première portion antérieure (114) comprend un stent antérieur (105c), le stent antérieur comprenant une pluralité d'unités de retenue, et
la deuxième portion antérieure (114') comprend une surface lisse dépourvue d'unités de retenue.

10. Dispositif d'annuloplastie selon la revendication 9, dans lequel le premier arc postérieur (113) comprend un premier stent postérieur (105a) comprenant une première pluralité d'unités de retenue, et
le deuxième arc postérieur (113') comprend un deuxième stent postérieur (105b) comprenant une deuxième pluralité d'unités de retenue s'étendant dans une direction allant vers la première pluralité d'unités de retenue.

11. Dispositif d'annuloplastie selon la revendication 1, dans lequel la première unité de retenue est incurvée vers la deuxième unité de retenue.

12. Dispositif d'annuloplastie selon la revendication 1, dans lequel les première et deuxième unités de retenue sont incurvées à l'opposé l'une de l'autre.

13. Dispositif d'annuloplastie selon l'une quelconque des revendications 1 à 12, dans lequel la quantité de courbure de la forme incurvée varie avec un angle (v₁, v₂) auquel les unités de retenue respectives sont positionnées autour d'une circonférence du stent.

14. Dispositif d'annuloplastie selon l'une quelconque des revendications 1 à 13, dans lequel la quantité de courbure de la forme incurvée varie avec une position (d₁, d₂) le long de la direction longitudinale (1) du stent à laquelle l'unité de retenue respective est positionnée.

15. Dispositif d'annuloplastie selon l'une quelconque des revendications 1 à 14, dans lequel les unités de retenue présentent une pointe crantée ou en forme de crochet (118).
